# EUROPEAN PATENT APPLICATION

(11) **EP 2 067 465 A2**
(43) Date of publication of application: **10.06.2009**
(21) Application number: 08170527.9
(22) Date of filing: 02.12.2008
(51) Int. Cl.: A61J 15/00, A61M 25/00

(54) **Reinforced enteral feeding catheter**

(30) Priority: 07.12.2007 US 952563
(71) Applicant: Moss, Gerald, White Plains NY 10605 (US)
(72) Inventor: Moss, Gerald, White Plains NY 10605 (US)
(74) Representative: Grey, Ian Michael

(57) **Abstract**

An enteral feeding catheter includes inner and outer layers of tubes and a spring positioned between them. The inner and outer tubes are coupled together so that the spring is held between them. The spring includes a helical wire and the distance between each adjacent coil is driven to equal the thickness of the wire. The combined thickness of the first and second tubes is less than or equal to the thickness of the wire.

## Description

### BACKGROUND OF THE INVENTION

### Field of the Invention

This invention relates to catheters.

### Description of the Related Art

Catheters, such as feeding and aspirating tubes, are generally inserted into a person's gastrointestinal tract through his or her nasal passage. Some examples of feeding and aspiration tubes are disclosed in U.S. Patent Nos. 3,618,613, 4,543,089, 4,642,092, 4,705,511, 4,806,182, 5,334,169, 5,599,325, 5,676,659, 5,947,940 and 6,508,804. Some catheters are dual lumen devices which include feeding and aspiration tubes. Examples of dual lumen devices are disclosed in U.S. Patent Nos. 5,807,311, 6,881,211 and 6,921,396.

The feeding tube is used to provide a patient with food internally and is often referred to as an enteral feeding catheter. Food is provided to nourish the patient, help him or her heal faster and resist infection, which decreases the patient recovery time. The aspirating tube is used to reduce abdominal distention, which occurs when swallowed air accumulates, and too much food is provided internally relative to its rate of absorption. Abdominal distention can impair the ability of the patient to breathe deeply and adequately cough to clear secretions. Abdominal distention can also cause discomfort and slow down the rate of bowel absorption. A slower rate of bowel absorption causes undernourishment and slows down the healing process, which increases the patient recovery time. Hence, it is desirable to reduce abdominal distention while providing the patient with sufficient nourishment to facilitate his or her recovery.

These feeding and aspirating tubes generally contact sensitive tissue, such as tissue in the nasal passage, or must penetrate healthy tissue to enter the stomach or intestine. Further, these feeding and aspirating tubes often have to reside within the nasal passage and gastrointestinal tract for a prolonged period of time. Hence, it is desirable to provide feeding and aspirating tubes which have a reduced wall thickness and still restrict the ability of the tube to kink. This allows more material to flow through the tube, without increasing patient discomfort.

### BRIEF SUMMARY OF THE INVENTION

The present invention employs a catheter which includes a spring positioned between inner and outer layers of resilient material. In one embodiment, the inner and outer layers of resilient material are in the form of inner and outer tubes, respectively, and the spring is a helical coil of wire. The inner and outer tubes are coupled together so that the spring is held between them. The spring restricts the ability of the inner and outer tubes to kink, and the combined thickness of the inner and outer layers is less than or equal to the diameter of the wire. In this way, the catheter is less bulky and is less likely to kink. The thinner walled catheter is more comfortable when it is used as an enteral feeding catheter. Catheters, in accordance with the invention, can be fabricated with a thickness less than about one-quarter the thickness of catheters currently available.

In one embodiment, the catheter includes first and second springs positioned between the inner and outer layers of resilient material. The first spring extends along a proximal portion of the catheter and the second spring extends along the distal portion. When the catheter is inserted into the patient, the proximal portion is within the nasal passage, and the distal portion extends into the stomach or beyond. The first and second springs can have a different number of turns per unit length. In some embodiments, the first spring has a larger number of turns per unit length then the second spring so that the catheter is less likely to kink when making the obligatory sharp bend in the patient's nasal passage. The distal portion of the catheter is not subjected to as severe bending forces, and the greater distance between turns of wire allow for easier cutting of orifices.

The present invention provides a method of manufacturing a catheter which includes providing inner and outer layers of resilient material and positioning a spring between them. The inner and outer layers of resilient material are in the form of inner and outer tubes, respectively, and the spring is a helical coil of wire. The inner and outer tubes are coupled together so that the spring is held between them. The inner and outer tubes can be coupled together in many different ways, such as by applying heat to them so they fuse together. In one embodiment of manufacturing the catheter, first and second springs are positioned between the inner and outer tubes, wherein the first spring extends along a proximal portion of the catheter and the second spring extends along the distal portion.

The present invention provides a method of using the catheter which includes inserting it through patient's nasal passage so it extends to the gastrointestinal tract. Alternatively, the catheter can be inserted through healthy tissue directly into the gastrointestinal tract.

These and other features, aspects, and advantages of the present invention will become better understood with reference to the following drawings and description.

### BRIEF DESCRIPTION OF THE DRAWINGS

FIG. 1 is a front view of a catheter, in accordance with the invention, inserted into a person.

FIG. 2a is a side view of the catheter of FIG. 1, which includes a spring positioned between inner and outer tubes.

FIG. 2b is a cut-away side view of a portion of the catheter of FIG. 2a.

FIG. 3 is a perspective end view of inner and outer tubes, as well as the spring, included with the catheter of FIG. 2a.

FIG. 4 is an end view of the spring included with the catheter of FIG. 2a.

FIG. 5 is an end view of the catheter of FIG. 2a, taken along a cut-line 5-5.

FIG. 6a is a partial cut-away view of the inner and outer tubes of FIG. 3 with the spring positioned between them.

FIG. 6b is a partial cut-away view of the inner and outer tubes of FIG. 6a coupled together so that the spring is held between them.

FIG. 7a is a side view of a catheter, in accordance with the invention, which includes two springs.

FIG. 7b is a cut-away side view of a portion of the catheter of FIG. 7a.

FIG. 7c is a cut-away side view of the proximal and distal portions of the catheter of FIG. 7a.

FIG. 8a is a perspective view of a dual lumen device which includes the catheter of FIG. 2a.

FIG. 8b is a close-up perspective view of the dual lumen device of FIG. 8a.

FIG. 9a is a perspective view of another dual lumen device which includes the catheter of FIG. 2a.

FIG. 9b is a close-up perspective view of the dual lumen device of FIG. 9a.

FIGS. 10a, 10b, 10c and 10d are flow diagrams of methods of manufacturing a catheter, in accordance with the invention.

FIGS. 11a and 11b are flow diagrams of methods of using a catheter, in accordance with the invention.

### DETAILED DESCRIPTION OF THE INVENTION

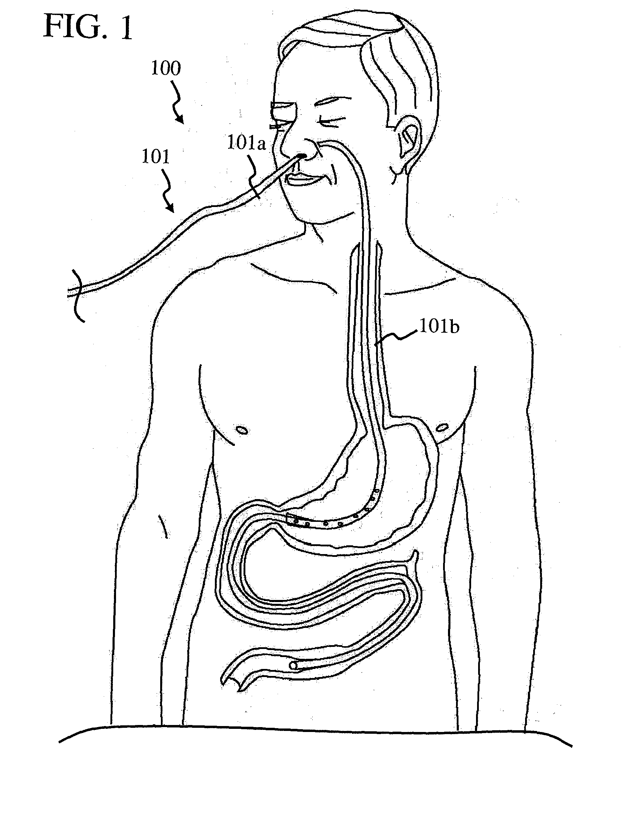

**FIG. 1** is a front view of a catheter 101, in accordance with the invention, inserted into a person 100. In this embodiment, catheter 101 extends through the nasal passage of person 100 and into the gastrointestinal tract. The proximal portion of catheter 101, denoted as portion 101a, is within the nasal passage of person 100. Further, the distal portion, denoted as portion 101b, is within the gastrointestinal tract of person 100. As discussed in more detail below, catheter 101 is less bulky and more resistant to kinking, so it is more comfortable for person 100 when it is inserted through his or her nasal passage and into the gastrointestinal tract. It should be noted that catheter 101 can be used as many different medical devices, such as a feeding tube, aspiration tube, etc. Further, catheter 101 includes a single lumen in this embodiment, but it generally includes one or more lumens.

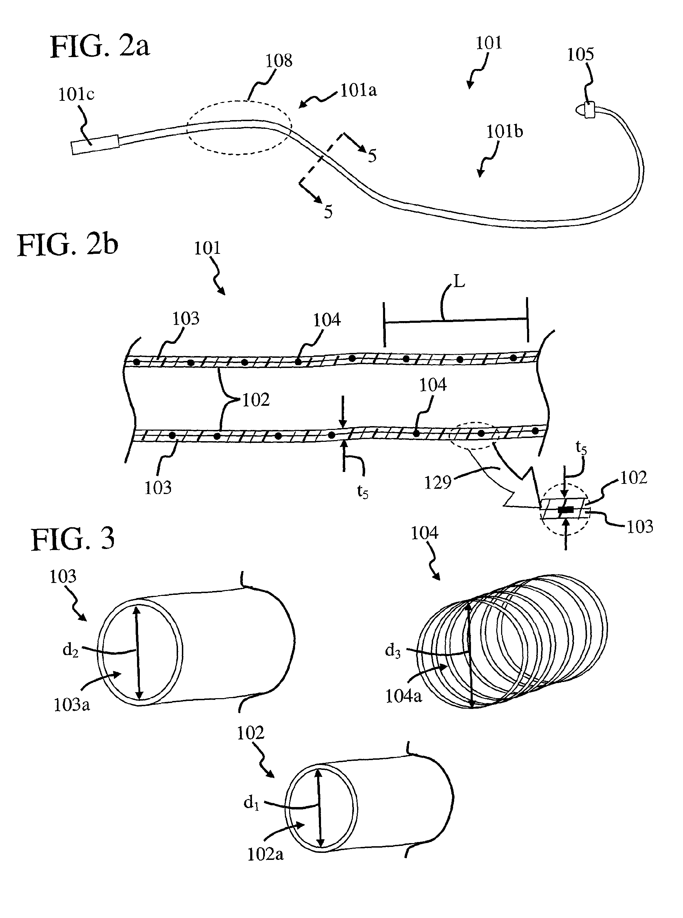

**FIG. 2a** is a side view of catheter 101, in accordance with the invention. In this embodiment, catheter 101 includes a connector 101c connected to proximal portion 101a and a tip 105. Connector 101c allows catheter 101 to be connected to a machine (not shown), such as a feeding or suction machine, and tip 105 retains distal portion 101b in the gastrointestinal tract.

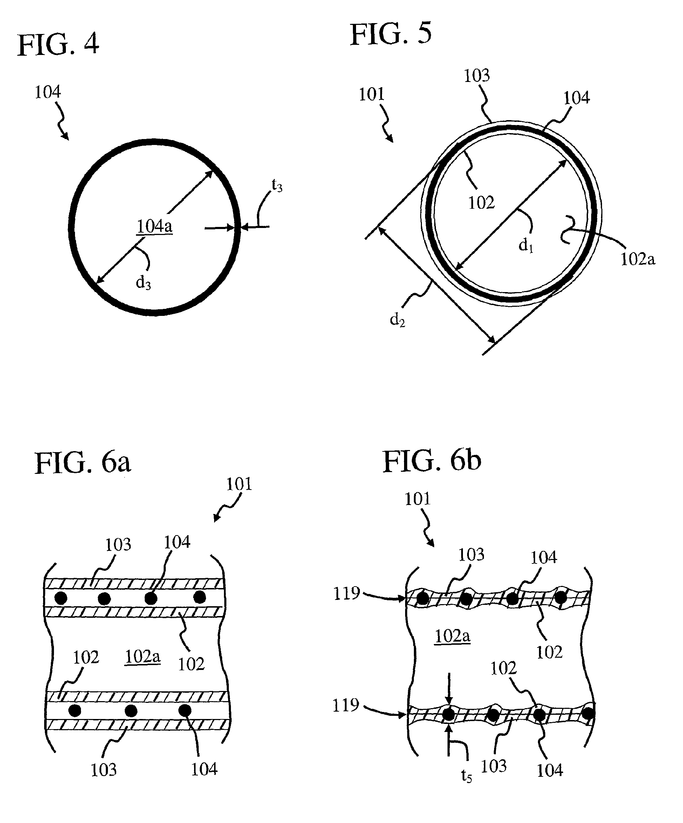

**FIG. 2b** is a cut-away side view of a portion of catheter 101, in accordance with the invention, as shown in a region 108 of **FIG. 2a****.** It should be noted that the portion of catheter 101 shown in **FIG. 2b** extends along its length and can correspond to proximal portion 101a and distal portion 101b. In accordance with the invention, catheter 101 includes an inner tube 102 and outer tube 103, as well as a spring 104, wherein inner and outer tubes 102 and 103 and spring 104 are shown in more detail in their perspective end views of **FIG. 3****.** More details of spring 104 are shown in its end view shown in **FIG. 4****,** and more details of catheter 101 are shown in its end view of **FIG. 5****,** which is taken along a cut-line 5-5 of **FIG. 2a****.**

In this embodiment, spring 104 extends through both proximal and distal portions 101a and 101b. In this way, catheter 101 includes one spring. Examples of springs which can be used in catheter 101 are disclosed in some of the patents referenced in the background of this application. The spring is generally helical in shape and includes a helical wire with multiple coils, wherein the helical wire is typically a round wire or a flat wire. When the helical wire is a round wire, it has a thickness that corresponds to its diameter because it has a circular cross-section. When the helical wire is a flat wire, it has a thickness that corresponds to its width because it has a rectangular cross-section. One example of spring 104 when it includes a flat wire is indicated by an indication arrow 129. The width of the flat wire extends radially outward from catheter 101, and the length of the flat wire extends transversely. It should be noted that, in this embodiment, the width of the flat wire is greater than its length.

Inner and outer tubes 102 and 103 can include many different types of resilient materials, but here they include biocompatible material that is flexible and elastic. The material can be of many different types, such as polyurethane, polysiloxane, and polyfluorohydrocarbons ("Teflon"). It should be noted that resilient materials are often referred to as elastomers. Examples of materials which can be used in catheter 101 are disclosed in some of the patents referenced in the background of this application. It should be noted that inner and outer tubes 102 and 103 generally include single layers of resilient material in the shape of a tube.

Inner and outer tubes 102 and 103 generally include the same materials. However, catheter 101 can be manufactured so that inner and outer tubes 102 and 103 include different materials. When inner and outer tubes 102 and 103 include the same material, they are more likely to be coupled together and to remain coupled together. When inner and outer tubes 102 and 103 include different materials, they are more likely to decouple from each other. The coupling and decoupling of materials is often referred to as lamination and delamination, respectively. More information regarding lamination and delamination is provided in U.S. Patent No. 4,806,182.

One reason inner and outer tubes 102 and 103 are used in catheter 101 is because they have a predetermined thickness, which is generally uniform along their lengths. Hence, the thickness of them combined together can be better controlled, so that the thickness of catheter 101 is better controlled. Further, inner and outer tubes 102 and 103 with desired thicknesses can be chosen before they are coupled together. In this way, the thickness of the combination of inner and outer tubes 102 and 103 can be chosen and better controlled. Other embodiments of catheters, such as those disclosed in U.S. Patent No. 3,618,613, include a coating. However, it is difficult to control the thickness and uniformity of the coating.

Catheter 101 has a wall thickness t₅, which can have many different values. In accordance with the invention, thickness t₅ is less than about 0.008 inches. In one embodiment, thickness t₅ is between about 0.008 inches and 0.001 inches. In another embodiment, thickness t₅ is between about 0.001 inches and 0.004 inches. In some embodiments, thickness t₅ is between about the thickness of spring 104 to about three times the thickness of spring 104. It should be noted that thickness t₅ generally varies along the length of catheter 101. For example, thickness t₅ is generally smaller between adjacent coils of spring 104 and larger proximate to the coils of spring 104.

Inner tube 102 has a diameter d₁ that is smaller than a diameter d₂ of outer tube 103 so that inner tube 102 will fit inside of outer tube 103. Further, spring 104 has a diameter d₃ that it larger than diameter d₁ of inner tube 102. Inner tube 102 is positioned within an opening 104a of spring 104, and inner tube 102 and spring 104 are positioned within an opening 103a of outer tube 103. Inner and outer tubes 102 and 103 are coupled together so that spring 104 is positioned between them, as shown in **FIG. 5****.** Inner and outer tubes 102 and 103 can be coupled together in many different ways, such as by heating them so they are laminated together. In some embodiments, the materials included in inner and outer tubes 102 and 103 intermix so that inner and outer tubes 102 and 103 are fused together.

Inner and outer tubes 102 and 103, as well as spring 104, can be manufactured in many different ways. For example, inner and outer tubes 102 and 103 can be manufactured by rolling flat pieces of resilient material into tubes. Inner and outer tubes 102 and 103 can also be extruded. Inner and outer tubes 102 and 103 can be extruded in many different ways, such as those disclosed in U.S. Patent Nos. 4,791,965, 4,888,146, 5,102,325, 5,542,937, 6,045,547, 6,165,166, 6,434,430, 6,692,804, 6,773,804 and 6,776,945. Spring 104 can be made in many different ways, such as those disclosed in U.S. Patent Nos. 4,302,959, 5,363,681, 6,006,572, 6,923,034 and 7,198,187.

**FIGS. 6a and 6b** are partial cut-away side views of catheter 101 showing one way it can be manufactured. **FIG. 6a** is a partial cut-away view of inner and outer tubes 102 and 103 with spring 104 positioned between them, and **FIG. 6b** is a partial cut-away view of inner and outer tubes 102 and 103 coupled together so that spring 104 is held between them. In this embodiment, and as shown in **FIG. 6a****,** inner and outer tubes 102 and 103 are separate pieces of material, wherein inner tube 102 is positioned within opening 104a of spring 104, and inner tube 102 and spring 104 are positioned within opening 103a of tube 103. Inner and outer tubes 102 and 103 are coupled together, as shown in **FIG. 6b****,** to form an interface 119 between them. Inner and outer tubes 102 and 103 are coupled together so that spring 104 is held between them adjacent to interface 119. Inner and outer tubes 102 and 103 can be coupled together in many different ways. In this embodiment, inner and outer tubes 102 and 103 are coupled together by applying heat to them so they are fused together. In this way, inner and outer tubes 102 and 103 are separate pieces and then coupled together to form a single integral piece which encloses spring 104. It should be noted that thickness t₅ in **FIG. 6b** varies along the length of catheter 101, wherein it has a smaller value between adjacent coils of spring 104 and a larger value proximate to the coils of spring 104.

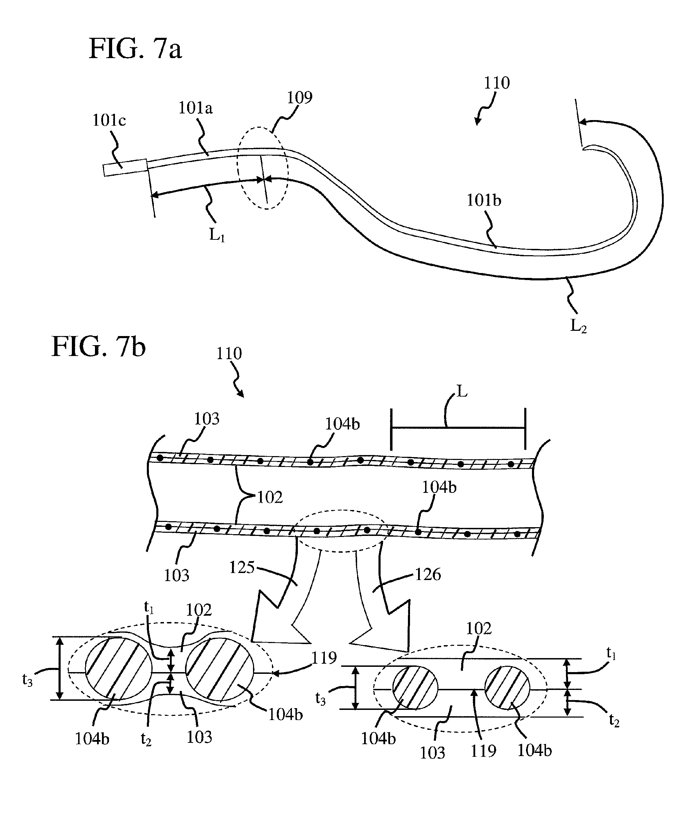

**FIG. 7a** is a side view of a catheter 110, in accordance with the invention. In this embodiment, proximal portion 101a extends a length L₁ along catheter 111 and distal portion 101b extends a length L₂ along catheter 111. Length L₁ can have many different length values, but it is generally between about six inches to about eighteen inches. Length L₂ can also have many different length values, but it is generally greater than length L₁. It should be noted that the length values of L₁ and L₂ typically depend on the size of patient 100. For example, lengths L₁ and L₂ are typically chosen so that it terminates within the gastrointestinal tract of person 100. In accordance with the invention, catheter 110 includes two springs, wherein one spring extends through proximal portion 101a and the other spring extends through distal portion 101b.

**FIG. 7b** is a cut-away side view of a portion of catheter 101 included in proximal portion 101a. It should be noted that the portion of catheter 101 shown in **FIG. 7b** extends along its length. In accordance with the invention, catheter 101 includes inner tube 102 and outer tube 103, as well as a spring 104b. Spring 104b can be the same or similar to spring 104 discussed in more detail above.

As indicated by an indication arrow 125, **FIG. 7b** shows a close-up view of inner and outer tubes 102 and 103, as well as spring 104b. Spring 104b is helical in shape and has a number of turns per unit length L, wherein unit length L typically corresponds to inches. Inner and outer tubes 102 and 103 have thicknesses denoted as thicknesses t₁ and t₂, respectively. Further, spring 104b includes a coil with a thickness t₃, which corresponds to its diameter in this example, since its cross-sectional shape is circular.

Thicknesses t₁, t₂ and t₃ can have many different values relative to each other. In accordance with the invention, thickness t₃ is greater than thickness t₁. Further, thickness t₃ is greater than thickness t₂. In the embodiment indicated by indication arrow 125, thickness t₃ is greater than the combination of thicknesses t₁ and t₂.

However, in other embodiments, such as the one indicated by an indication arrow 126 in **FIG. 7b**, the combination of thicknesses t₁ and t₂ is greater than or equal to thickness t₃. In one particular embodiment, the combination of thicknesses t₁ and t₂ is between about two to three times greater than thickness t₃.

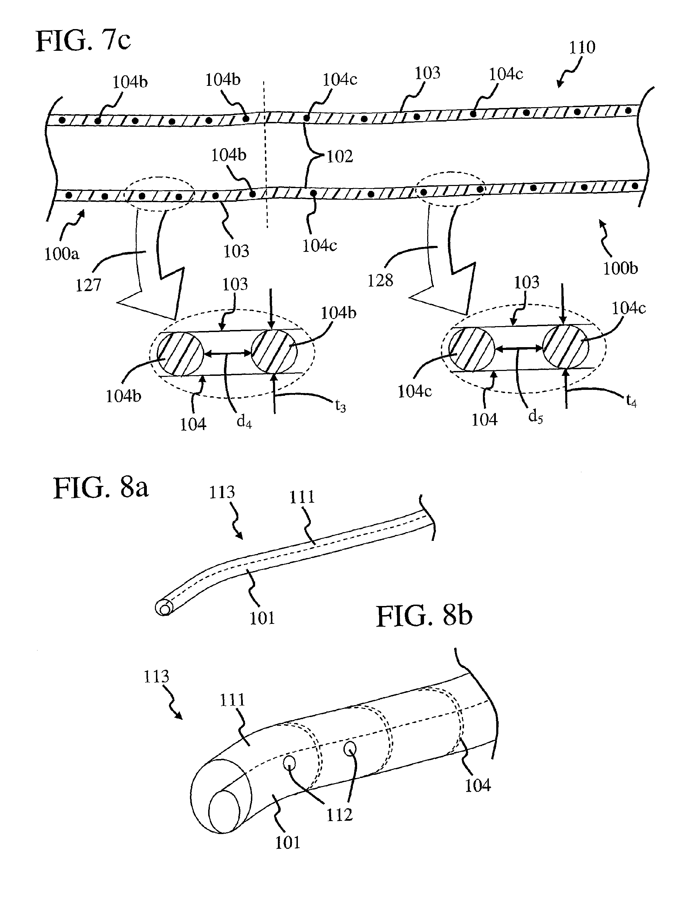

**FIG. 7c** is a cut-away side view of proximal and distal portions 101a and 101b in a transition region 109 of **FIG. 7a****.** In this embodiment, portions 101a and 101b include springs 104b and 104c, respectively, positioned between inner and outer tubes 102 and 103. Springs 104b and 104c both include helical wires having thicknesses t₃ and t₄, respectively. In general, a catheter in accordance with the invention includes one or more springs. For example, in the embodiment shown in **FIG. 2a****,** the catheter shown includes one spring and, in the embodiment shown in **FIG. 7a****,** the catheter shown includes two springs.

In accordance with the invention, the helical wire of spring 104b includes a number of turns per unit length that is different from the number of turns per unit length of the helical wire of spring 104c. In this embodiment, the number of turns per unit length L of spring 104b is greater than the number of turns per unit length L of spring 104c. It should be noted that springs 104b and 104c can be the same or similar to spring 104, which is discussed in more detail above.

As indicated by an indication arrow 127, **FIG. 7c** shows a close-up view of portion 101a of catheter 101. In this embodiment, the turns of spring 104b are spaced apart from each adjacent turn by a distance d₄. Distance d₄ can have many different values, but, in one embodiment, distance d₄ is driven to equal thickness t₃. However, in general, distance d₄ is less than or equal to thickness t₃.

As indicated by an indication arrow 128, **FIG. 7c** shows a close-up view of portion 101b of catheter 101. In this embodiment, the turns of spring 104c are spaced apart from each adjacent turn by a distance d₅. Distance d₅ can have many different values, but, in one embodiment, distance d₅ is greater than distance d₄. Distance d₅ is also typically greater than or equal to distance t₄. Thicknesses t₃ and t₄ can have many different values. In some embodiments, thicknesses t₃ and t₄ are equal to each other and, in other embodiments, thickness t₃ is less than thickness t₄. In still other embodiments, thickness t₃ is greater than thickness t₄.

Catheter 101 can be inserted into patient 100 in many different ways. In this embodiment, length L₁ **(****FIG. 7a****)** is chosen so that portion 101a is within the nasal passage of person 100 and length L₂ **(****FIG. 7a****)** is chosen so that portion 101b is within the gastrointestinal tract of person 100. Spring 104b is chosen to have a number of turns per unit length L and a thickness t₃ so that it is restricted from kinking when it extends through the nasal passage of person 100. Distance d₄ is also chosen to restrict the ability of portion 101a to kink in the nasal passage of person 100. Further, springs 104a and 104b are chosen to reduce the likelihood of portions 101a and 101b from collapsing when extending through person 100.

**FIG. 8a** is a perspective view of a dual lumen device 113, in accordance with the invention. More information regarding dual lumen devices can be found in U.S. Patent Application No. 11/838,657, entitled "DUAL LUMEN GASTROINTESTINAL FEEDING AND ASPIRATING DEVICE", filed on August 14, 2007 by Kurt W. Grathwohl, the contents of which are incorporated herein by reference. In this embodiment, dual lumen device 113 includes an aspiration tube 111 and catheter 101, wherein catheter 101 extends through aspiration tube 111. Hence, aspiration tube 111 extends around catheter 101.

In accordance with the invention, catheter 101 includes inner and outer tubes 102 and 103 with a spring positioned between them, as discussed in more detail above. In some embodiments, the inner periphery of aspiration tube 111 is attached to outer tube 103 and, other embodiments, catheter 101 extends through aspiration tube 111, but it is not attached to it. In some embodiments, aspiration tube 111 is thicker than the combination of inner and outer tubes 102 and 103 so that it protects them from being damaged.

**FIG. 8b** is a close-up perspective view of dual lumen device 113, wherein aspiration tube 111 includes spring 104 and inner and outer tubes 102 and 103, wherein spring 104 is positioned between inner and outer tubes 102 and 103. Aspiration tube 111 can be of many different types, such as those disclosed in U.S. Patent Application No. 11/838,657. However, in some embodiment, aspiration tube 111 can have the same or a similar configuration as catheter 101. For example, aspiration tube 111 can include inner and outer tubes with a spring positioned therebetween, as shown in **FIG. 3****.** Further, in some embodiments, aspiration tube 111 can include inner and outer tubes with first and second springs positioned therebetween, as shown in **FIG. 7c****.**

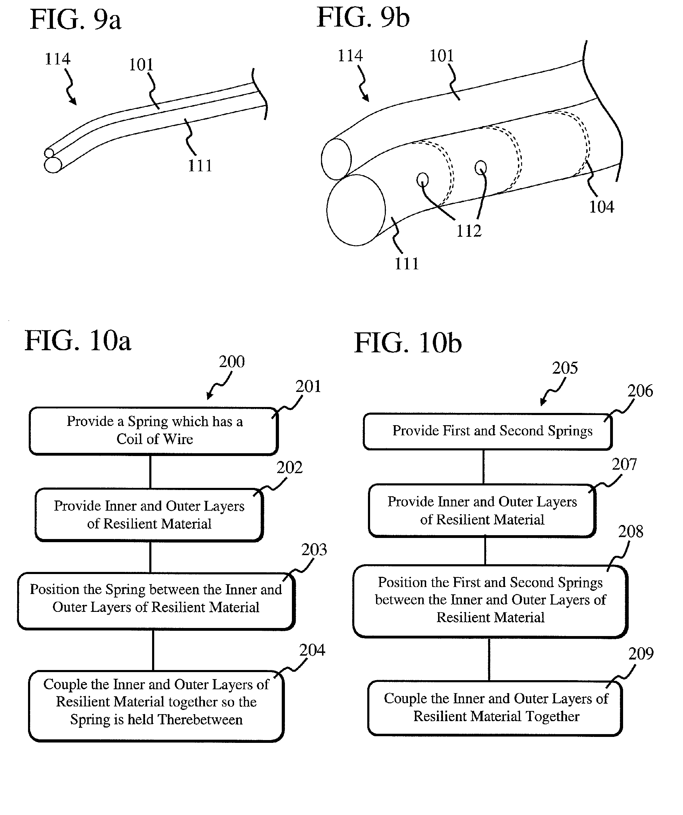

**FIG. 9a** is a perspective view of a dual lumen device 114, in accordance with the invention. It should be noted that dual lumen device 114 can include the same features of dual lumen device 113. In this embodiment, dual lumen device 114 includes catheter 101 attached to and carried by an aspiration tube 111, wherein catheter 101 operates as a feeding tube. In accordance with the invention, catheter 101 includes inner and outer tubes 102 and 103 with a spring positioned therebetween, as discussed in more detail above, wherein the outer periphery of aspiration tube 111 is attached to outer tube 103.

It should be noted that dual lumen devices 113 and 114 are shown here as being a single integrated piece, but they can be separate pieces in other embodiments. For example, catheter 101 and aspiration tube 111 can be separate pieces coupled together. In other embodiments, dual lumen device 114 is a single tube having separate feeding and aspiration channels extending through it. More information regarding feeding and aspiration tubes can be found in U.S. Patent Nos. 4,543,089 and 4,642,092 by Moss, both of which are incorporated herein by reference.

In accordance with the invention, aspiration tube 111 also operates as a stent for the insertion of an instrument into a patient, as discussed in more detail in U.S. Patent Application No. 11/838,657. The instrument can be of many different types, but it is generally a medical instrument, such as a gastroscope. The instrument is elongated so its distal end can be moved through aspiration tube 111 between locations internal and external to the patient.

**FIG. 9b** is a close-up perspective view of dual lumen device 114, wherein aspiration tube 111 includes spring 104 and inner and outer tubes 102 and 103, wherein spring 104 is positioned between inner and outer tubes 102 and 103. Aspiration device 111 generally includes opposed orifices and/or windows, as described in more detail in U.S. Patent Application No. 11/838,657. In this embodiment, dual lumen device 114 includes orifices 112, wherein orifices 112 extend through aspiration tube 111 between adjacent coils of spring 104.

**FIG. 10a** is a flow diagram of a method 200 of manufacturing a catheter, in accordance with the invention. In this embodiment, method 200 includes a step 201 of providing a spring having a coil of wire. It should be noted that the coil of wire can be in many different forms, but here it is a helical coil of wire. Method 200 includes a step 202 of providing inner and outer layers of resilient material. The inner tube has a smaller diameter than the outer tube so that the inner tube will fit within the outer tube. In accordance with the invention, the first and second layers of resilient material each have a combined thickness that is less than the thickness of the wire included with the spring.

The inner and outer layers of material are generally in the form of inner and outer tubes, respectively. The inner and outer layers of resilient material can be provided to the user in the form of tubes (i.e. preformed), or they can be formed into tubes by the user, such as by extrusion. Further, the spring can be provided to the user preformed or it can be formed by the user. In some embodiments, the inner and outer tubes include the same material, but, in other embodiments, the inner and outer tubes include different materials. In some embodiments, the inner and outer tubes include single layers of material, but, in other embodiments, the inner and outer tubes include multiple layers or materials.

Method 200 includes a step 203 of positioning the spring between the inner and outer layers of resilient material. Method 200 includes a step 204 of coupling the inner and outer layers of resilient material together so the spring is held therebetween. Portions of the inner and outer layers of resilient material between the coils of the helical wire are typically coupled together. The inner and outer layers of resilient material can be coupled together in many different ways, such as by applying heat to them so they fuse together.

**FIG. 10b** is a flow diagram of a method 205, in accordance with the invention, of manufacturing a feeding tube. In this embodiment, method 205 includes a step 206 of providing first and second springs, which can be of many different types. In this embodiment, the first and second springs include helical coils of wire. In some embodiments, the number of turns per unit length of the first and second springs are different. For example, the first spring can have a number of turns per unit length that is greater than the number of turns per unit length of the second spring. In some embodiments, the distance between each adjacent coil in the first spring is driven to equal the diameter of the wire of the first spring.

Method 205 includes a step 207 of providing inner and outer layers of resilient material. The inner and outer layers of resilient material are generally in the form of inner and outer tubes, respectively, wherein the diameter of the inner tube is less than the diameter of the outer tube. Method 205 includes a step 208 of positioning the first and second springs between the inner and outer layers of resilient material, and a step 209 of coupling the inner and outer layers of resilient material together so the first and second springs are held between them. In this embodiment, the first spring extends along a proximal portion of the feeding tube and the second spring extends along the distal portion.

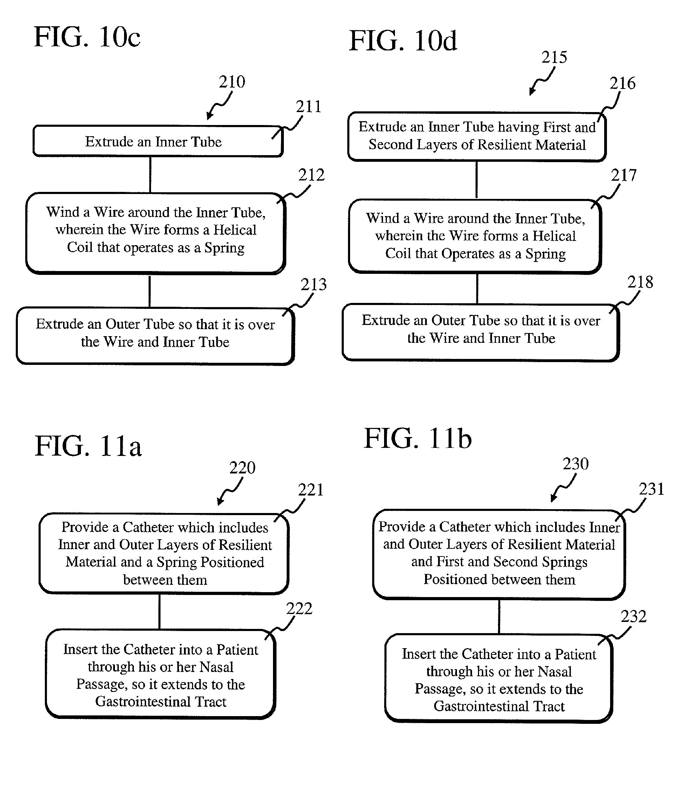

**FIG. 10c** is a flow diagram of a method 210 of manufacturing a catheter, in accordance with the invention. In this embodiment, method 210 includes a step 211 of extruding an inner tube and a step 212 of winding a wire around the inner tube, wherein the wire forms a helical coil that operates as a spring. Method 210 includes a step 213 of extruding an outer tube so that it is over the wire and inner tube. While portions of the inner and outer tubes are being extruded, the portions of the inner and outer tubes that have been extruded are fused together so that the spring is held between them. It should be noted that the spring is typically wound around the inner tube in a controlled manner so that the number of turns per unit length is controlled. For example, the number of turns per unit length can be adjusted as the inner tube is extruded. The number of turns per unit length can be adjusted by increasing or decreasing its value.

**FIG. 10d** is a flow diagram of a method 215 of manufacturing a catheter, in accordance with the invention. In this embodiment, method 215 includes a step 216 of extruding an inner tube having first and second layers of resilient material. In one embodiment, the first layer of material includes Teflon and the second layer of material includes another material, such as polyurethane. In general, the first layer of material has less friction than the second layer of material so that an instrument can slide through the catheter easier. Method 215 includes a step 217 of winding a wire around the inner tube, wherein the wire forms a helical coil that operates as a spring. In this embodiment, the wire is adjacent to the second layer of material. Method 215 includes a step 218 of extruding an outer tube so that it is over the wire and inner tube. While portions of the inner and outer tubes are being extruded, the portions of the inner and outer tubes that have been extruded are fused together so that the spring is held between them.

**FIG. 11a** is a flow diagram of a method 220 of using a catheter, in accordance with the invention. In this embodiment, method 220 includes a step 221 of providing a catheter which includes inner and outer layers of resilient material and a spring positioned between them. The inner and outer layers of resilient material are generally in the form of inner and outer tubes, respectively, wherein the inner tube can fit inside the outer tube. It should be noted that the catheter generally includes one or more lumens. Further, in some embodiments, the catheter can be included in a dual lumen device. Method 220 includes a step 222 of inserting the catheter into a patient through his or her nasal passage, so it extends to the gastrointestinal tract.

**FIG. 11b** is a flow diagram of a method 230 of using a catheter, in accordance with the invention. In this embodiment, method 230 includes a step 231 of providing a catheter which includes inner and outer layers of resilient material and first and second springs positioned between them. The first and second springs can be of many different types, such as helical coils of wire. In some embodiments, the number of turns per unit length of the first and second springs are different. For example, the first spring can have a number of turns per unit length that is greater than the number of turns per unit length of the second spring. In some embodiments, the distance between each adjacent coil in the first spring is driven to equal the diameter of the wire of the first spring. In this embodiment, the first spring extends along a proximal portion of the feeding tube and the second spring extends along the distal portion.

Method 230 includes a step 232 of inserting the catheter into a patient through his or her nasal passage, so it extends to the gastrointestinal tract. The catheter is positioned so that its proximal portion is adjacent to the patient's nasal passage and the distal portion extends through the patient's gastrointestinal tract. In this way, the first spring is adjacent to the patient's nasal passage and the second spring is not. The length of the catheter is chosen so that the proximal portion extends through the patient's nasal passage and the distal portion extends into the patient's gastrointestinal tract.

It should be noted that the steps in the methods disclosed herein can be carried out in many different orders. It should also be noted that the catheters of the methods disclosed herein generally include one or more lumens. Further, in some embodiments, the catheters can be included in a dual lumen device. For example, the catheter can be attached to and carried by an aspiration tube, wherein the aspiration tube can include inner and outer layers of resilient material with a spring positioned between them. The methods disclosed herein can include one or more of the steps disclosed in the methods described in U.S. Patent Application No. 11/838,657.

The embodiments of the invention described herein are exemplary and numerous modifications, variations and rearrangements can be readily envisioned to achieve substantially equivalent results, all of which are intended to be embraced within the spirit and scope of the invention.

## Claims

1. A catheter, comprising inner and outer layers of resilient material; and a first spring positioned between the inner and outer layers, the first spring including a helical coil of wire.

2. A catheter according to claim 1, wherein the combined thickness of the inner and outer layers is less than about 0.203mm (0.008 inches).

3. The catheter of claim 1 or 2, wherein the combined thickness of the inner and outer layers is less than about three times the thickness of the first spring.

4. The catheter of any preceding claim, wherein the distance between adjacent coils is driven to equal the thickness of the wire.

5. The catheter of any preceding claim, further including a second spring positioned between the inner and outer layers, the second spring including a helical coil of wire.

6. The catheter of claim 5, wherein the first spring extends along a proximal portion of the catheter and the second spring extends along a distal portion of the catheter.

7. The catheter of claim 5 or 6, wherein the number of turns per unit length of the first spring is more than the number of turns per unit length of the second spring.

8. The catheter of any of claims 5 to 7, wherein the inner and outer layers are coupled together between coils of the first and second springs.

9. The catheter of claim 8, wherein the thickness of the portions of the first and second layers coupled together is less then the thickness of the wire of the first spring.

10. The catheter of any preceding claim, wherein the inner and outer layers of resilient material are in the form of inner and outer tubes.

11. A method of manufacturing a catheter, comprising providing a first spring having a helical coil of wire; extruding inner and outer layers of resilient material so that the first spring is positioned between them; and coupling the inner and outer layers of resilient material together so the first spring is held therebetween.

12. The method of claim 11, wherein the combined thickness of the inner and outer layers of resilient material is less than about 0.203mm (0.008 inches).

13. The method of claim 11 or 12, wherein the distance between each adjacent coil in the first spring is driven to equal the thickness of the wire along the proximal portion of the catheter.

14. The method of any of claims 11 to 13, further including providing a second spring having a helical coil of wire, wherein the inner and outer layers of resilient material are extruded so that the second spring is positioned between them.

15. The method of claim 14, further including positioning the first and second springs so they extend along proximal and distal portions, respectively, of the catheter.
